# EUROPÄISCHE PATENTSCHRIFT

(11) **EP 1 774 913 B1**
(45) Veröffentlichungstag und Bekanntmachung des Hinweises auf die Patenterteilung: **07.01.2009**
(21) Anmeldenummer: 06018914.9
(22) Anmeldetag: 09.09.2006
(51) Int. Cl.: A61B 17/00, G02B 23/24

(54) **Endoskop**
Endoscope
Endoscope

(30) Priorität: 11.10.2005 DE 102005049021
(43) Veröffentlichungstag der Anmeldung: 18.04.2007
(73) Patentinhaber: Richard Wolf GmbH, 75438 Knittlingen (DE)
(72) Erfinder: Schlagenhauf, Frank, Dipl.-Ing., 75245 Neulingen (DE); Diener, Jörg, 75038 Oberderdingen (DE)
(74) Vertreter: Hemmer, Arnd

(56) Entgegenhaltungen:
- GB-A- 1 452 185
- US-A- 5 406 940
- US-A- 5 685 820
- US-B1- 6 419 626

## Beschreibung

Die Erfindung betrifft ein Endoskop oder Technoskop.

Es kommt insbesondere in der Industrie häufig vor, dass Bauteile, beispielsweise Gussteile geprüft und im Notfall nachbearbeitet werden müssen, z. B. Querbohrungen in Pumpengehäusen oder Kugelkanäle in Motorblöcken. Während und nach der Nachbearbeitung sind weitere Prüfungen erforderlich, um festzustellen, ob die Nacharbeitung erfolgreich war. Dies erfordert einen häufigen Wechsel der Werkzeuge und Instrumente, welche während Prüfung und Nachbearbeitung eingesetzt werden.

US 6,419,626 B1 sowie GB 1 452 185 A offenbaren Zangen, welche im Inneren eine Optik bzw. einen Bildsensor enthalten, mit welchen eine visuelle Beobachtung des axial vor dem distalen Ende der Zange gelegenen Bereiches möglich ist.

US 5,406,940 offenbart ein chirurgisches Instrument, welches einen Hohlschaft aufweist, an dessen distalen Ende eine axiale Verlängerung mit seitlichen, d.h. in Umfangsrichtung gerichteten Schneiden ausgebildet ist. Durch oszillierende Bewegung des Schaftes in Umfangsrichtung kann mit dem Schneiden Gewebe geschnitten werden. Dieses Instrument eignet sich nur für sehr spezielle Anwendungsfälle, insbesondere können Bearbeitungen von härteren Materialien als Körpergewebe, wie sie in technischen Anwendungen auftreten können mit diesem Instrument nicht durchgeführt werden.

Ferner hat das US 5,406,940 bekannte Instrument den Nachteil, dass die Schneiden und die Endoskopoptik so angeordnet sind, dass der Bereich, in welchem die Schneiden wirken, nicht unmittelbar im Blickfeld der Endoskopoptik liegt. Insofern ist mit diesem Instrument eine Bearbeitung und gleichzeitige Prüfung nur schwer möglich, da dazu das Instrument zwischen einer Bearbeitungs- und einer Beobachtungsposition gedreht werden muss, um ein- und dieselbe Stelle betrachten und bearbeiten zu können.

US 5,685,820 offenbart ein Instrument zum Penetrieren von Körpergewebe in Form eines Trokars. Dieser ist an einer feststehenden Spitze transparent ausgebildet, sodass ein Durchdringen des Körpergewebes unter visueller Kontrolle möglich ist.

Es ist Aufgabe der Erfindung, ein Endoskop oder Technoskop bereitzustellen, welches eine vereinfachte Prüfung und Bearbeitung von Werkstücken ermöglicht.

Diese Aufgabe wird durch ein Endoskop mit den im Anspruch 1 angegebenen Merkmalen gelöst. Bevorzugte Ausführungsformen ergeben sich aus den Unteransprüchen.

Auch wenn das erfindungsgemäße Endoskop nachfolgend vorrangig zum Einsatz in technischen Anwendungen beschrieben wird, kann das erfindungsgemäße Endoskop auch im Bereich der Human- und Veterinärmedizin sowie der Zahnmedizin zum Einsatz kommen.

Das erfindungsgemäße Endoskop weist an seinem distalen Ende ein Bearbeitungswerkzeug auf. Mit diesem Bearbeitungswerkzeug können die gewünschten Bearbeitungen bzw. Nachbearbeitungen eines Werkstückes durchgeführt werden. Dazu ist das Bearbeitungswerkzeug beweglich bzw. bewegbar ausgebildet. Zum Antrieb bzw. zur Bewegung des Bearbeitungswerkzeuges ist ein sich in Längsrichtung des Endoskops, d. h. vom proximalen zum distalen Ende erstreckender beweglicher Hohlschaft vorgesehen. Über die Bewegung des Hohlschaftes wird das Bearbeitungswerkzeug in Bewegung versetzt bzw. angetrieben. Erfindungsgemäß ist ferner im Inneren des beweglichen Hohlschaftes eine vorzugsweise feststehende Endoskopoptik angeordnet. Über diese Endoskopoptik kann der distalseitig des Endoskops liegende bzw. den das distale Ende des Endoskops umgebende Raum beobachtet werden, wie es von herkömmlichen Endoskopen her bekannt ist. Die Endoskopoptik kann feststehen, d. h. fest mit einem Endoskopgehäuse verbunden sein. Alternativ kann die Endoskopoptik auch drehbar ausgebildet sein, um durch Drehung der Endoskopoptik das Blickfeld der Endoskopoptik in dem zu untersuchenden Hohlraum zu bewegen. Jedoch ist die Endoskopoptik erfindungsgemäß in der Weise feststehend, dass sie zum Antrieb des Bearbeitungswerkzeuges nicht gemeinsam mit dem Hohlschaft bewegt wird. Durch die erfindungsgemäße Kombination eines beweglichen Bearbeitungswerkzeuges und einer Endoskopoptik ist es gleichzeitig möglich, eine optische Kontrolle und eine Bearbeitung des Werkstückes vorzunehmen. Aufgrund der konzentrischen Anordnung des Hohlschaftes und der Endoskopoptik ist darüber hinaus ein sehr kompakter Aufbau des Endoskops mit sehr kleinem Durchmesser möglich, so dass auch sehr kleine Hohlräume inspiziert und insbesondere unter visueller Kontrolle bearbeitet werden können.

Am distalen Ende des Endoskops sind in der Wandung des Hohlschaftes Ausnehmungen ausgebildet, welche im Blickfeld der Endoskopoptik liegen oder in das Blickfeld der Endoskopoptik bewegbar sind. D.h. das Sichtfenster der Endoskopoptik am distalen Ende ist vorzugsweise so ausgerichtet, dass es der Ausnehmung zugewandt ist. Diese Anordnung ermöglicht es, dass von der Endoskopoptik aus eine Beobachtung durch die Wandung des Hohlschaftes, nämlich durch die Ausnehmung hindurch möglich ist. Je nach Bewegungsart und Richtung des Hohlschaftes ist es nicht möglich, eine Ausnehmung so auszugestalten, dass über die gesamte Bewegungsbahn des Hohlschaftes die Ausnehmung im Blickfeld der Endoskopoptik liegt, so dass eine kontinuierliche Beobachtung durch die Ausnehmung hindurch möglich ist. Dies ist insbesondere bei rotierender Ausgestaltung des Bearbeitungswerkzeuges und des Hohlschaftes der Fall. Daher sind mehrere Ausnehmungen vorgesehen, welche so angeordnet sind, dass sie bei Bewegung des Hohlschaftes bzw. des Bearbeitungswerkzeuges das Blickfeld der Endoskopoptik periodisch überstreichen. Bei schneller Bewegung, insbesondere schneller Rotation des Bearbeitungswerkzeuges oder des Hohlschaftes ermöglicht dies eine kontinuierliche Beobachtung mittels der Endoskopoptik, da die Trägheit des Auges die Unterbrechungen des Blickfeldes durch die Wandung des Hohlschaftes bzw. durch massive Teile des Bearbeitungswerkzeuges nicht wahrnehmen wird.

Je nach Anordnung und Ausgestaltung des Bearbeitungswerkzeuges am distalen Ende des Hohlschaftes können die Ausnehmungen in der Wandung des Hohlschaftes und/oder im Bearbeitungswerkzeug angeordnet sein.

Ferner ist das Blickfeld der Endoskopoptik zumindest teilweise in radialer Richtung bezüglich der Längsachse des Endoskops gerichtet. Das bedeutet, die Endoskopoptik ermöglicht eine Beobachtung in radialer Richtung bzw. im Winkel zur Längsachse des Endoskops durch die Umfangswandung des Hohlschaftes hindurch, wenn eine Ausnehmung im Blickfeld der Endoskopoptik liegt bzw. das Blickfeld der Endoskopoptik bei Bewegung des Hohlschaftes überstreicht.

Vorzugsweise ist der Hohlschaft in Längsrichtung des Endoskops beweglich und/oder um seine Längsachse drehbar. Durch die Bewegung in Längsrichtung kann eine oszillierende Bewegung des Bearbeitungswerkzeuges in Längsrichtung des Endoskops erreicht werden. Durch Rotation um die Längsachse des Hohlschaftes kann eine drehende Bewegung des Bearbeitungswerkzeuges erreicht werden. Die Rotation des Hohlschaftes und damit auch die Rotation des Bearbeitungswerkzeuges erfolgt vorzugsweise derart, dass eine Drehung von mehr als 360° in einer Drehrichtung, vorzugsweise eine kontinuierliche Rotation um die Längsachse in derselben Drehrichtung vollführt wird. In speziellen Ausführungsformen ist es auch möglich, beide Bewegungen zu überlagern, so dass das Bearbeitungswerkzeug gleichzeitig oszilliert und rotiert. Ferner ist es nicht zwingend erforderlich, dass das Bearbeitungswerkzeug die gleiche Bewegungsart wie der Hohlschaft ausführt, vielmehr kann hier beispielsweise auch eine Umsetzung einer oszillierenden Bewegung des Hohlschaftes in eine Rotationsbewegung, beispielsweise eine oszillierend rotierende Bewegung des Bearbeitungswerkzeuges erreicht werden.

Vorzugsweise weist der Hohlschaft einen kreisförmigen Querschnitt auf. Entsprechend weist die Endoskopoptik im Inneren des Hohlschaftes weiter bevorzugt einen korrespondierenden Querschnitt auf, so dass eine kompakte konzentrische Anordnung des Hohlschaftes und der Endoskopoptik möglich ist. Der kreisförmige Querschnitt des Hohlschaftes bietet sich insbesondere bei rotierender Bewegung des Hohlschaftes um seine Längsachse an.

Zweckmäßigerweise sind zwischen einer Innenwandung des Hohlschaftes und einer Außenwandung der Endoskopoptik Lagerelemente angeordnet. Diese Lagerelemente stellen eine präzise Führung bzw. Lagerung des rotierenden Hohlschaftes an der feststehenden Endoskopoptik sicher. Dazu sind vorzugsweise zumindest am proximalen und am distalen Ende Lagerelemente zwischen Endoskopoptik und Hohlschaft vorgesehen. Durch die Lagerelemente wird eine gleichbleibende definierte Ausrichtung der Endoskopoptik zu dem Hohlschaft sichergestellt, so dass eine präzise Beobachtung der Bearbeitung durch das Bearbeitungswerkzeug mittels der Endoskopoptik stets gewährleistet ist.

Am proximalen Ende des Endoskops ist weiter bevorzugt ein Antrieb zur Bewegung des Hohlschaftes vorgesehen. Dies kann beispielsweise ein Elektromotor sein. Alternativ kann am proximalen Ende eine Kupplung vorgesehen sein, an welche ein standardisierter Antrieb, beispielsweise ein Elektromotor bzw. Hohlwellenmotor oder eine Welle zur Verbindung mit einem externen Antrieb angekuppelt werden kann.

Gemäß einer besonderen Ausführungsform der Erfindung kann der Hohlschaft flexibel ausgebildet sein. So kann der Hohlschaft als flexible Welle ausgebildet sein, so dass eine Bearbeitung auch schwer zugänglicher Räume oder Stellen mittels des Endoskops möglich ist. Dabei ist die flexible Ausgestaltung des Hohlschaftes so, dass seine Bewegung zum Antrieb des Bearbeitungswerkzeuges, insbesondere eine Rotation, weiterhin möglich ist.

Entsprechend kann auch die Endoskopoptik flexibel ausgebildet sein, um in einem flexiblen Hohlschaft angeordnet zu werden. So kann die Endoskopoptik gemeinsam mit dem flexiblen Hohlschaft gebogen werden bzw. Biegungen erfahren, um schwer zugängliche Stellen zu bearbeiten und optisch zu prüfen.

Gemäß einer weiteren bevorzugten Ausführungsform ist am distalen Ende des Hohlschaftes eine Kupplung zur Aufnahme des Bearbeitungswerkzeuges ausgebildet. Eine solche Kupplung ermöglicht es, verschiedene Bearbeitungswerkzeuge mit dem Hohlschaft, vorzugsweise austauschbar zu verbinden. So kann das Bearbeitungswerkzeug je nach auszuführender Bearbeitung ausgewählt und mit dem Hohlschaft verbunden werden. Ferner ist es möglich, das Bearbeitungswerkzeug bei Verschleiß austauschen zu können.

Vorzugsweise ist am distalen Ende des Hohlschaftes ein Getriebe, insbesondere ein Winkeltrieb angeordnet, mittels welchem das Bearbeitungswerkzeug bewegbar ist. Ein solches Getriebe ermöglicht zum einen eine Über- oder Untersetzung der Bewegung des Hohlschaftes zur Bewegung des Bearbeitungswerkzeuges. Ferner kann die Bewegungsrichtung geändert werden, so kann beispielsweise eine linear oszillierende Bewegung des Hohlschaftes in eine Rotationsbewegung, insbesondere eine oszillierende Rotationsbewegung des Bearbeitungswerkzeuges umgesetzt werden. Alternativ ist es auch möglich, eine Dreh- bzw. Rotationsbewegung des Hohlschaftes über das Getriebe in eine oszillierende Linearbewegung des Bearbeitungswerkzeuges umzusetzen, beispielsweise über ein Exentergetriebe oder einen Nockentrieb. Ferner kann das Getriebe als Winkeltrieb ausgebildet sein, so dass es möglich ist, dass die Drehachse des Bearbeitungswerkzeuges abgewinkelt zur Dreh- bzw. Längsachse des Hohlschaftes verläuft. Beispielsweise kann die Drehachse des Bearbeitungswerkzeuges um 90° abgewinkelt zu der Drehachse des Hohlschaftes verlaufen. Dies kann beispielsweise durch ein Stirn- oder Kegelradgetriebe erreicht werden. Dabei wird das an dem Hohlschaft angeordnete Zahn- bzw. Kegelrad in seinem Zentrum vorzugsweise hohl ausgebildet, so dass sich die Endoskopoptik durch diesen Bereich hindurch erstrecken kann oder zumindest das Blickfeld der Endoskopoptik durch diesen Bereich hindurch verlaufen kann. So ist eine Beobachtung des distalen Endes durch das Getriebe hindurch möglich.

Um die Beobachtungsrichtung bzw. das Blickfeld der Endoskopoptik dem gewünschten Einsatzzweck anzupassen, kann auch das Sichtfenster der Endoskopoptik am distalen Ende gewinkelt angeordnet werden, so dass die Blickrichtung nicht in distaler Richtung, sondern gewinkelt zur Längsachse des Endoskops beispielsweise um 45° in radialer Richtung erfolgt. Auch kann das Sichtfenster so angeordnet werden, dass das Blickfeld in radialer Richtung gerichtet ist, d. h. die optische Achse des Blickfeldes normal zur Längsachse des Endoskops bzw. zur Längsachse des Hohlschaftes verläuft. Entsprechend kann am distalen Ende der Endoskopoptik ein Prisma oder anderes geeignetes optisches Element zur entsprechenden Umlenkung des Strahlenganges angeordnet sein.

Gemäß einer weiteren bevorzugten Ausführungsform ist an dem Hohlschaft zumindest ein sich in Längsrichtung des Endoskop erstreckender Sondenkanal ausgebildet. Dabei ist dieser Sondenkanal besonders bevorzugt so ausgebildet, dass er fest mit dem Hohlschaft verbunden ist, d. h. sich gemeinsam mit dem Hohlschaft bewegt. Durch diesen Sondenkanal ist es möglich, Bearbeitungswerkzeuge zu führen oder zu halten. So kann der Sondenkanal zum distalen Ende des Hohlschaftes hin geöffnet sein und in den Sondenkanal beispielsweise ein Schmirgeldraht eingesetzt sein, welcher gemeinsam mit dem Hohlschaft rotiert. Wenn sich der Sondenkanal bis zum proximalen Ende erstreckt, ist es ferner möglich, das Bearbeitungswerkzeug, beispielsweise einen Schmirgeldraht bei Abnutzung vom proxmalen Ende her nachzuführen. Alternativ kann ein stab- oder drahtförmiges Bearbeitungswerkzeug in dem Sondenkanal auch gemeinsam mit dem Hohlschaft in Längsrichtung des Endoskops oszillierend bewegt werden, um eine gewünschte Bearbeitung distalseitig des Endoskops bzw. im Bereich des distalen Endes des Endoskops an einem Werkstück vornehmen zu können. Auch hier ist es möglich, dass das Bearbeitungswerkzeug vom proximalen Ende her nachgeführt wird, wenn sich der Sondenkanal bis zum proximalen Ende erstreckt. Weiter bevorzugt ist es auch möglich, mehr als einen Sondenkanal an dem Hohlschaft vorzusehen. Die Sondenkanäle sind vorzugsweise im Bereich der Umfangswandung des Hohlschaftes ausgebildet, d. h. sie erstrecken sich exzentrisch zu dem Hohlschaft.

Gemäß einer weiteren bevorzugten Ausführungsform ist das Bearbeitungswerkzeug direkt am distalen Ende des Hohlschaftes ausgebildet. Insbesondere kann die Umfangswandung des Hohlschaftes am distalen Ende selber das Bearbeitungswerkzeug bilden. Dieser Bereich des Hohlschaftes kann beispielsweise speziell geschliffen oder auf andere Weise geeignet ausgebildet sein, um ein Fräs- oder Schleifwerkzeug zu bilden. Hier kann das Material des Hohlschaftes auch entsprechend gehärtet oder beschichtet sein, beispielsweise mit Hartmetall, Keramik, Diamant oder ähnlichen. Auch die Form des distalen Endes des Hohlschaftes kann an die Bearbeitungsaufgabe angepasst sein, beispielsweise ring- oder kegelförmig ausgebildet sein. Hier sind nahezu alle von Bearbeitungswerkzeugen insbesondere zur spanenden Bearbeitung bekannten Formen und Beschichtungen einsetzbar. So ist es beispielsweise möglich, den Hohlschaft am distalen Ende umfänglich mit Diamant zu beschichten, um bei Rotation oder Oszillation dieser beschichteten Fläche eine Schleifbearbeitung von Werkstücken durchführen zu können.

Beispielsweise kann das Bearbeitungswerkzeug als Säge-, Fräser- und/oder Schleifwerkzeug ausgebildet sein. Es ist somit insbesondere möglich, das Bearbeitungswerkzeug für alle Arten der spanenden Bearbeitung, sei es mit geometrisch bestimmter oder unbestimmter Schneide, auszubilden.

Gemäß einer weiteren bevorzugten Ausführungsform ist der Hohlschaft abnehmbar bzw. austauschbar ausgebildet. Dies ermöglicht es zum einen, das gesamte Endoskop zu Reinigungs- und Wartungszwecken zu zerlegen. Vorzugsweise kann auch die Endoskopoptik abnehmbar bzw. austauschbar ausgebildet sein. Zum anderen ermöglicht diese Ausgestaltung, den Hohlschaft austauschen zu können, beispielsweise bei Beschädigung oder um verschiedene Hohlschäfte mit ein und demselben Endoskop verbinden zu können. So können verschiedene Hohlschäfte für verschiedene Bearbeitungsaufgaben vorgesehen sein, welche dann je nach Wunsch mit dem Endoskop verbunden werden können. Beispielsweise ist es auch möglich wahlweise einen flexiblen oder starren Endoskopschaft bzw. Hohlschaft mit zugehöriger Endoskopoptik vorzusehen. Ferner kann der Hohlschaft, sofern das Bearbeitungswerkzeug direkt an seinem distalen Ende ausgebildet oder fest mit diesem distalen Ende verbunden ist, als Wegwerfteil ausgebildet werden, welcher bei Verschleiß des Bearbeitungswerkzeuges komplett ersetzt wird durch einen neuen Hohlschaft mit neuem Bearbeitungswerkzeug.

Die Endoskopoptik kann grundsätzlich wie jede bekannte Endoskopoptik ausgebildet sein. Dies können beispielsweise ein Linsensystem, eine Faseroptik oder auch ein Kamerasystem sein, welche eine Bildübertragung vom distalen Ende des Endoskops zum proximalen Ende bzw. zu entfernteren Anzeigemitteln ermöglichen. Bei einem Kamerasystem ist es insbesondere bevorzugt, die Kamera unmittelbar im Bereich des distalen Endes anzuordnen und eine Bildübertragung über elektrische Signale vorzusehen, wobei sich dann entsprechende Anschlussleitungen durch einen Schaft zum proximalen Ende des Endoskops erstrecken. Dieser ist dann als feststehender, d. h. sich bei der Bearbeitung nicht gemeinsam mit dem Hohlschaft bewegender Optikschaft, welcher die Endoskopoptik bildet, ausgebildet.

In dem Fall, dass die Bilderfassung über die Endoskopoptik mittels einer Kamera erfolgt, ist es bevorzugt, dass diese Bilderfassung über die Kamera mit der Bewegung des Hohlschaftes synchronisiert ist. Das bedeutet, dass in dem Fall, dass eine oder mehrere Ausnehmungen im Bearbeitungswerkzeug oder der Wandung des Hohlschaftes vorgesehen sind, welche das Blickfeld der Kamera bzw. der Endoskopoptik periodisch überstreichen, die Verschlusszeiten der Kamera so synchronisiert oder programmiert werden, dass die Kamera immer genau dann ein Bild erfasst, wenn eine Ausnehmung das Blickfeld der Kamera bzw. der Endoskopoptik überstreicht. Bei genügend schneller Bewegung des Hohlschaftes bzw. des Bearbeitungswerkzeuges und entsprechend synchronisierter Kamera wird so ein für das menschliche Auge konstantes Bild aufgenommen.

Gemäß einer weiteren bevorzugten Ausführungsform ist zwischen der Außenwandung der Endoskopoptik und der Innenwandung des Hohlschaftes zumindest ein Spülkanal ausgebildet. Dieser Spülkanal ist vorzugsweise konzentrisch zu dem Hohlschaft und der Endoskopoptik und ermöglicht es, vom proximalen Ende des Endoskops her ein Fluid, beispielsweise ein Gas oder eine Spülflüssigkeit zuzuführen, um den Bearbeitungsraum, in welchem sich das Bearbeitungswerkzeug bewegt von Verunreinigungen und insbesondere von Spänen, welche das Bearbeitungswerkzeug erzeugt, zu befreien. So kann insbesondere auch das Sichtfenster der Endoskopoptik am distalen Ende durch ein Spülfluid, beispielsweise eine Spülflüssigkeit oder ein Spülgas, frei von Verunreinigungen gehalten werden.

Gemäß einer besonderen Ausgestaltung ist es möglich, zwischen Endoskopoptik und Hohlschaft mehrere Spülkanäle anzuordnen. Dazu kann beispielsweise eine weitere Hülse zwischen Endoskopoptik und Hohlschaft derart angeordnet werden, dass zwischen der Hülse und der Endoskopoptik ein Freiraum und zwischen der Hülse und der Innenwandung des Hohlschaftes ein zweiter Freiraum verbleibt. So werden zwei ineinander liegende, vorzugsweise konzentrische Kanäle geschaffen. So kann durch einen dieser Kanäle beispielsweise eine Spülflüssigkeit zu- und durch den anderen Kanal abgeführt werden. Diese zusätzliche Hülse bzw. Zwischenwandung ist vorzugsweise relativ zu der Endoskopoptik ortfest ausgebildet, kann jedoch auch ortsfest zu dem Hohlschaft ausgebildet sein, so dass sie sich gemeinsam mit dem Hohlschaft bewegt. Letztere Ausgestaltung ermöglicht es insbesondere, eine Spülflüssigkeit immer an definierter Stelle des Bearbeitungswerkzeuges zu- oder abzuführen unabhängig von der momentanen Position des Bearbeitungswerkzeuges auf seiner Bewegungsbahn, insbesondere der aktuellen Winkelposition des Bearbeitungswerkzeuges. Anstatt die Spülkanäle konzentrisch, d. h. als Ringkanäle auszubilden, ist es auch möglich, an der Endoskopoptik oder dem Hohlschaft exzentrisch zur Längsachse des Endoskops angeordnete Spülkanäle vorzusehen.

Gemäß einer weiteren bevorzugten Ausführungsform ist eine Lichthülse zur Beleuchtung eines das distale Ende des Endoskops umgebenden Raumes vorgesehen. Dies ist insbesondere der Raum, welcher im Blickfeld der Endoskopoptik liegt. Dazu weist die Lichthülse zweckmäßigerweise eine distalwärts oder in Richtung des Blickfeldes gerichtete Abstrahlfläche auf.

Die Lichthülse ist dabei vorzugsweise außerhalb des Hohlschaftes angeordnet. Alternativ ist es jedoch auch möglich, die Lichthülse im Inneren des Hohlschaftes beispielsweise an der Endoskopoptik anzuordnen. Die Lichthülse kann vorzugsweise als Lichtleiter ausgebildet sein. Bei dieser Ausgestaltung ist die eigentliche Lichtquelle, d. h. das Leuchtmittel vorzugsweise am proximalen Ende des Endoskops angeordnet oder ankuppelbar. Dies ermöglicht eine sehr schlanke Ausgestaltung des gesamten Endoskops an seinem distalen Ende, da die Lichthülse bzw. deren Lichtleiter sehr dünn ausgebildet werden können. Alternativ oder zusätzlich ist es auch möglich, zumindest ein Leuchtmittel direkt in der Lichthülse anzuordnen. Als Leuchtmittel können besonders bevorzugt Leuchtdioden Verwendung finden, welche sehr klein ausgebildet werden können und darüber hinaus nur geringe Abwärme erzeugen.

Die Lichthülse ist vorzugsweise mit ringförmigem Querschnitt ausgebildet, so dass sie konzentrisch zu der Endoskopoptik und/oder dem Hohlschaft angeordnet werden kann. Dies begünstigt einen sehr kleinen Durchmesser des gesamten Endoskopschaftes. Darüber hinaus kann so eine gleichmäßige Ausleuchtung des distalseitigen Raumes des Endoskops erreicht werden.

Die Lichthülse kann gemeinsam mit dem Hohlschaft beweglich ausgebildet sein, d. h. die Lichthülse rotiert beispielsweise gemeinsam mit dem Hohlschaft. Dies macht in dem Fall, dass elektrische Leuchtmittel in der Lichthülse angeordnet sind, Schleifkontakte zur Energieübertragung erforderlich, welche vorzugsweise im Bereich des proximalen Endes des Endoskops angeordnet werden. In dem Fall, dass die Lichthülse als reiner Lichtleiter ausgebildet ist, wird, vorzugsweise am proximalen Ende des Endoskops, ein Lichtanschluss vorgesehen, welcher eine Rotation oder lineare Bewegung der Lichthülse relativ zu einer feststehenden Lichtquelle bzw. einem feststehenden Lichtleiter ermöglicht. So kann beispielsweise die proximale Stirnfläche der Lichthülse einer parallelen Austrittsfläche eines feststehenden Lichtleiters gegenüberliegen, so dass Licht von dem feststehenden Lichtleiter auf die sich bewegende Lichthülse übertragen werden kann.

Alternativ ist es auch möglich, die Lichthülse relativ zu dem Hohlschaft beweglich anzuordnen. Dies ermöglicht es, die Lichthülse relativ zu Endoskopoptik und den übrigen Bauteilen des Endoskops feststehend auszubilden, so dass sich der Hohlschaft sowohl relativ zu der Lichthülse als auch der Endoskopoptik bewegen kann.

Gemäß einer weiteren bevorzugten Ausführungsform ist der Hohlschaft umfänglich von einer Schutzhülse umgeben. Diese Schutzhülse kann sicherstellen, dass keine Körperteile oder Werkstückteile, welche nicht bearbeitet werden sollen, mit dem sich bewegenden Hohlschaft in Kontakt kommen. So kann die Schutzhülse oder eine weitere Schutzhülse auch das Bearbeitungswerkzeug in einem Teilbereich, beispielsweise in einem Umfangssegment umgeben, um zu verhindern, dass nicht zu bearbeitende Bereiche des Werkstückes mit dem Bearbeitungswerkzeug in Kontakt kommen können.

Nachfolgend wird die Erfindung beispielhaft anhand der beigefügten Figuren beschrieben. In diesen zeigt:
- Fig. 1: eine teilweise geschnittene Ansicht eines Endoskops gemäß einer ersten Ausführungsform der Erfindung,
- Fig. 2: eine teilweise geschnittene Ansicht eines flexiblen Endoskops gemäß der Erfindung,
- Fig. 3: eine Schnittansicht eines Endoskopschaftes gemäß einer weiteren Ausführungsform der Erfindung,
- Fig. 4: eine Schnittansicht eines Endoskopschaftes gemäß einer weiteren Ausführungsform der Erfindung,
- Fig. 5: eine Schnittansicht eines Endoskopschaftes gemäß einer weiteren Ausführungsform der Erfindung,
- Fig. 6: eine Schnittansicht eines Endoskopschaftes gemäß einer weiteren Ausführungsform der Erfindung,
- Fig. 7: eine teilweise geschnittene Ansicht eines Endoskopschaftes gemäß einer weiteren Ausführungsform der Erfindung,
- Fig. 8: das distale Ende eines Endoskopschaftes gemäß einer weiteren Ausführungsform der Erfindung,
- Fig. 9: eine Schnittansicht eines distalen Endes eines Endoskopschaftes gemäß einer weiteren Ausführungsform der Erfindung,
- Fig. 10: eine Schnittansicht des distalen Endes eines Endoskopschafts gemäß einer weiteren Ausführungsform der Erfindung,
- Fig. 11: eine teilweise geschnittene Ansicht eines Endoskopschaftes gemäß einer weiteren Ausführungsform der Erfindung,
- Fig. 12: eine Schnittansicht eines Endoskopschaftes gemäß einer weiteren Ausführungsform der Erfindung,
- Fig. 13: eine Schnittansicht eines Endoskopschaftes gemäß einer weiteren Ausführungsform der Erfindung und
- Fig. 14: eine Schnittansicht des distalen Endes eines Endoskopschaftes gemäß einer weiteren Ausführungsform der Erfindung.

In nachfolgender Beschreibung werden verschiedene Ausführungsbeispiele der Erfindung beschrieben. Dabei werden gleiche Bauteile mit gleichen Bezugsziffern bezeichnet. Ferner werden im Wesentlichen nur die Unterschiede zwischen den einzelnen Ausführungsbeispielen beschrieben. Es ist dabei zu verstehen, dass Einzelheiten, welche bei nachfolgenden Ausführungsbeispielen nicht näher beschrieben werden, entsprechend der Beschreibung zu den anderen Ausführungsbeispielen ausgestaltet werden können.

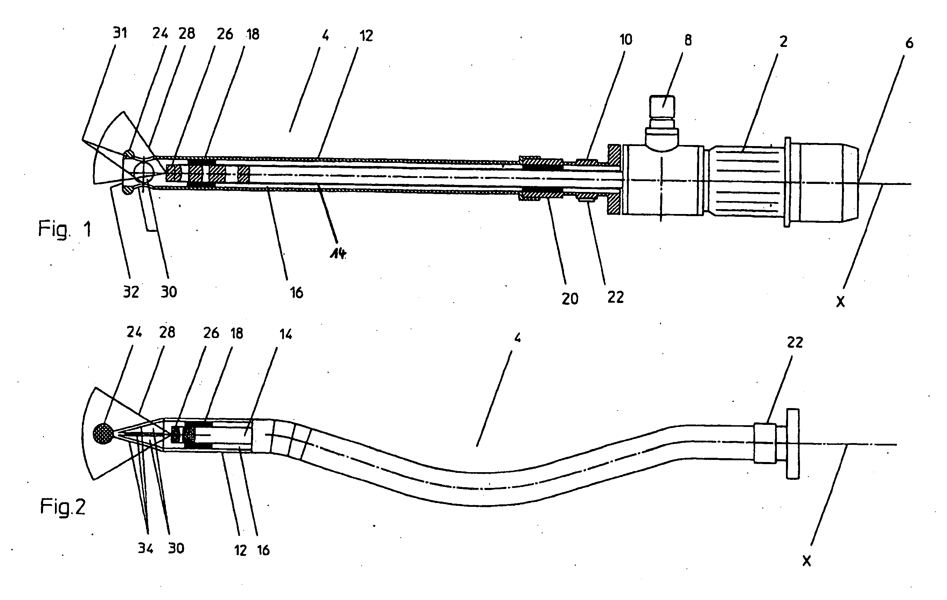

Anhand von Fig. 1 wird zunächst der grundsätzliche Aufbau des erfindungsgemäßen Endoskops erläutert. Das erfindungsgemäße Endoskop besteht aus einem Endoskopgehäuse 2 und einem sich von diesem ausgehend distalwärts in Richtung der Längsachse X erstreckende Endoskopschaft 4. Das Endoskopgehäuse 2 ist im Wesentlichen wie bei bekannten Endoskopen ausgestaltet und weist eine Einblicköffnung 6 am proximalen Ende ggf. einen Lichtanschluss 8 sowie einen Anschluss für in Fig. 1 nicht gezeigten Drehantrieb 10 auf. Anstelle der Einblicköffnung 6 kann bei Verwendung einer Videooptik auch ein elektrischer Anschluss für ein externes Anzeigemittel, insbesondere einen Monitor bzw. Videocontroller vorgesehen sein.

Erfindungswesentlich ist die Ausgestaltung des Endoskopschaftes 4, wobei es sich bei dem in Fig. 1 gezeigten Endoskopschaft 4 um einen starren Endoskopschaft 4 handelt, welcher sich gerade erstreckt. Der Endoskopschaft 4 ist vorzugsweise lösbar mit dem Endoskopgehäuse 2 verbunden. Dies ermöglicht es, verschiedene Endoskopschäfte 4 an ein und dasselbe Endoskopgehäuse 2 anzusetzen, um das Endoskop für verschiedene Einsatzzwecke verwenden zu können. Ferner ermöglicht es, Einzelteile bei Verschleiß auszutauschen und begünstigt die Reinigung und Wartung des Endoskops.

Der Endoskopschaft 4 wird in dem in Fig. 1 gezeigten Beispiel von einem äußeren Hohlschaft 12 und einer zentral im Inneren des Hohlschaftes 12 angeordneten Endoskopoptik 14 gebildet. Hohlschaft 12 und Endoskopoptik 14 erstrecken sich parallel und konzentrisch zu der Längsachse X. Die Endoskopoptik 14 ist wie bekannte Endoskopoptiken im gezeigten Beispiel als Linsenoptik ausgebildet. Die optischen Elemente sind bei dieser Endoskopoptik in einem starren Schaft angeordnet. Zwischen dem Hohlschaft 12 und der Endoskopoptik 14 ist ein Freiraum 16 in Form eines Ringraumes gebildet, welcher als Spülkanal dienen kann. Im Freiraum 16 ist im Bereich des distalen Endes des Endoskops ein Lagerelement 18 und im Bereich des proximalen Endes des Endoskopschaftes 4 ein Lagerelement 20 angeordnet. Die Lagerelemente 18 und 20 sind als Gleitlager ausgebildet und ermöglichen eine bewegliche Führung des Hohlschaftes 12 am Außenumfang der Endoskopoptik 14. Diese Führung ist vorzugsweise im Wesentlichen spielfrei. Bei Verwendung des Freiraumes 16 als Spülkanal können in den Lagerelementen 18 und 20 Fluiddurchgänge in Richtung parallel zu der Längsachse X ausgebildet sein. Im gezeigten Beispiel ist der Hohlschaft 12 drehbar um die Endoskopoptik 12 angeordnet. Die Drehung erfolgt über den Antrieb 10 an einem am Außenumfang des Hohlschaftes 12 ausgebildeten Ritzel 22 im Bereich des proximalen Endes des Hohlschaftes 12. Das distale Ende des Hohlschaftes 12 bildet ein Bearbeitungswerkzeug 24. Im gezeigten Beispiel erweitert sich das distale Ende 24, so dass ein ringförmiger Wulst gebildet ist. Dieser kann an seiner Außenumfangsfläche beispielsweise mit Diamanten beschichtet sein, um ein Schleifwerkzeug zu bilden. So können mit dem Bearbeitungswerkzeug 24 bei Rotation des Hohlschaftes 12 Schleifbearbeitungen durchgeführt werden.

Die Endoskopoptik ist im gezeigten Beispiel so angeordnet, dass das Sichtfenster 26 am distalen Ende der Endoskopoptik 14 gegenüber dem distalen Ende des Hohlschaftes 12 in proximaler Richtung zurückversetzt ist. So ist das Sichtfenster 26 geschützt im Inneren des Hohlschaftes 12 angeordnet. Im gezeigten Beispiel ist das Blickfeld 28 abgewinkelt zur Längsachse X der Endoskopoptik 14 ausgebildet, d. h. das Blickfeld 28 ist nicht genau in distaler Richtung, sondern etwas in radialer Richtung verschwenkt angeordnet. Dies kann beispielsweise durch ein Prisma im distalen Ende der Endoskopoptik 14 erreicht werden.

Beabstandet vom distalen Ende des Hohlschaftes 12 distalseitig des Sichtfensters 26 sind in der Umfangswandung des Hohlschaftes 12 Ausnehmungen 30 ausgebildet. Diese Ausnehmungen 30 ermöglichen gemeinsam mit der offen ausgebildeten distalen Stirnfläche 32 des Hohlschaftes 12 eine Beobachtung des das Bearbeitungswerkzeug 24 umgebenden Bereiches. So kann durch die Endoskopoptik 14, durch die Ausnehmungen 30 und die Stirnfläche 32 hindurch gesehen werden, um den Bereich des Werkstückes, welcher gleichzeitig durch das Bearbeitungswerkzeug 24 bearbeitet werden kann, zu beobachten. Damit kann eine Bearbeitung unter ständiger visueller Kontrolle erreicht werden. Zwischen den umfänglich verteilt angeordneten Ausnehmungen 30 verbleiben in der Umfangswandung des Hohlschaftes 12 Stege 31. Aufgrund der schnellen Rotation des Hohlschaftes 12 um die Längsachse X bei der Bearbeitung beeinträchtigen diese das Bild, welches der Beobachter durch die Endoskopoptik 14 sieht, jedoch nicht. Aufgrund der Trägheit des Auges wird der Beobachter diese Stege 31, welche periodisch das Blickfeld 28 passieren, nicht wahrnehmen.

Durch den Freiraum 16 kann beispielsweise ein Gas oder Fluid vom proximalen Ende her zugeführt werden, um den Bearbeitungsraum am distalen Ende von Verunreinigungen frei zu spülen, insbesondere Späne, welche von dem Bearbeitungswerkzeug 24 erzeugt werden, wegzuspülen, so dass das Sichtfenster 26 und das Blickfeld 28 frei von Verunreinigungen gehalten werden.

Fig. 2 zeigt eine zweite Ausführungsform eines Endoskopschaftes 4. Das Endoskopgehäuse 2 ist hier nicht gezeigt, jedoch entsprechend der vorangehenden Beschreibung ausgebildet. Dies gilt auch für die weiteren nachfolgend beschriebenen Ausführungsformen, bei welchen lediglich der Endoskopschaft 4 gezeigt ist.

Der Endoskopschaft 4 gemäß Fig. 2 ist flexibel ausgebildet, d. h. die Längsachse X kann je nach Bedarf gekrümmt bzw. gebogen werden und verläuft nicht zwingend gerade. Dies ermöglicht die Bearbeitung und Beobachtung auch schwer zugänglicher Stellen, insbesondere ermöglicht es, den Endoskopschaft 4 auch in gekrümmte Öffnungen und Kanäle einzuführen. Um die Flexibilität des Endoskopschaftes 4 zu realisieren, ist der Hohlschaft 12 als flexible Hohlwelle und die Endoskopoptik 14 ebenfalls flexibel, beispielsweise als flexible Faseroptik ausgebildet.

Ein weiterer Unterschied der Ausführungsform gemäß Fig. 2 zu der Ausführungsform gemäß Fig. 1 ist die Ausbildung des Bearbeitungswerkzeuges 24. Das Bearbeitungswerkzeug 24 in Fig. 2 ist als kugelförmiger Fräser- bzw. Schleifkopf ausgebildet, welcher über Stege 34 mit dem distalen Ende des Hohlschaftes 12 verbunden ist. Die Stege 34 verlaufen in kegel- bzw. pyramidenförmiger Form, so dass sie an der proximalen Seite des kugelförmigen Kopfes zusammentreffen. Zwischen den Stegen 34 sind umfänglich Freiräume bzw. Ausnehmungen 30 gebildet, welche wie anhand von Fig. 1 beschrieben, den Blick durch das Bearbeitungswerkzeug 24 auf den das Bearbeitungswerkzeug 24 umgebenden Raum, ermöglichen. Bei dem in Fig. 2 gezeigten Beispiel ist das Blickfeld 28 konzentrisch zur Längsachse X in distaler Richtung ausgerichtet.

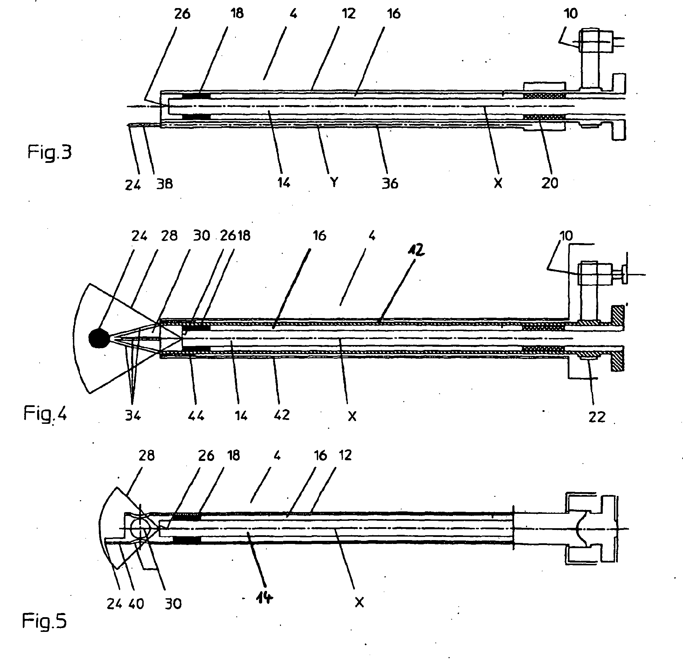

Fig. 3 zeigt eine weitere Variante des Bearbeitungswerkzeuges. Gemäß Fig. 3 ist an dem Hohlschaft 12 ein sich parallel zu der Längsachse X an der Umfangswandung des Hohlschaftes 12 erstreckender Sondenkanal 36 ausgebildet. In den Sondenkanal 36, dessen Längsachse Y parallel zu der Längsachse X des Endoskopschaftes 4 verläuft, ist ein Schmirgeldraht 38 eingesetzt, welcher sich über das distale Ende des Hohlschaftes 12 und des Sondenkanals 36 hinaus erstreckt. D. h. der Schmirgeldraht 38 steht am distalen Ende von dem Endoskopschaft 4 in distaler Richtung vor. Bei Rotation des Hohlschaftes 12 rotiert der Schmirgeldraht 18 auf einer Kreisbahn und bildet so das Bearbeitungswerkzeug 24, welches beispielsweise zum Abschleifen bzw. Abschlagen von Graten eingesetzt werden kann. Die Endoskopoptik 14 weist wie vorangehend beschrieben ein distalwärts gerichtetes Sichtfenster 26 auf und ermöglicht einen freien Blick durch die distale Öffnung des Hohlschaftes 12 auf den Bereich, in welchem die Bearbeitung durch den Schmirgeldraht 38 vorgenommen wird. Auch hier ist das Sichtfenster 26 proximalwärts gegenüber dem distalen Ende des Hohlschaftes 12 zurückversetzt.

In Fig. 3 und 4 ist ferner der Antrieb 10 als Riementrieb, welcher das Ritzel 22 antreibt, dargestellt. Hier kann ein Zahnriemen Verwendung finden. Alternativ kann auch ein anderer Treibriemen Verwendung finden, wobei dann kein Ritzel 22, sondern an entsprechender Stelle ein glattes Treibrad beispielsweise für einen Keilriemen Verwendung findet. Auch beliebig andere Arten eines entsprechenden Drehantriebes des Hohlschaftes 12 können hier Verwendung finden.

Eine Ausführungsform ähnlich zu der in Fig. 3 ist in Fig. 5 gezeigt. Hier ist anstatt eines Sondenkanals 36 ein distalwärts gerichteter Vorsprung 40 vorgesehen, welcher einen sich in distaler Richtung erstreckenden Stift bzw. Steg bildet, welcher z. B. oszillierend angetrieben wird und so ein Bearbeitungswerkzeug 24 bildet. Dazu kann der Vorsprung 40 beispielsweise diamantbeschichtet sein. In dem Ausführungsbeispiel gemäß Fig. 5 sind ferner Ausnehmungen 30, wie anhand von Fig. 1 beschrieben, vorgesehen.

Fig. 4 zeigt ein Bearbeitungswerkzeug entsprechend dem anhand von Fig. 2 beschriebenen Bearbeitungswerkzeug 24. Im Unterschied zu Fig. 2 ist der Endoskopschaft 4 bei dem Ausführungsbeispiel gemäß Fig. 4 starr ausgebildet. Zusätzlich ist hier eine Schutzhülse 42 vorgesehen, welche den Hohlschaft 12 umfänglich mit Abstand umgibt. Die Schutzhülse 42 ist feststehend ausgebildet, d. h. sie rotiert nicht gemeinsam mit dem Hohlschaft 12. Entsprechend ist am distalen und proximalen Ende ein Lagerelement 44 als Gleitlager zwischen dem rotierenden Hohlschaft 12 und der feststehenden Schutzhülse 42 ausgebildet. Die Schutzhülse 42 verhindert, dass der rotierende bzw. sich bewegende Hohlschaft 12 mit Körper- oder Werkstückteilen in Berührung kommt und diese beschädigt. Ferner wird die Verletzungsgefahr für eine Bedienperson minimiert.

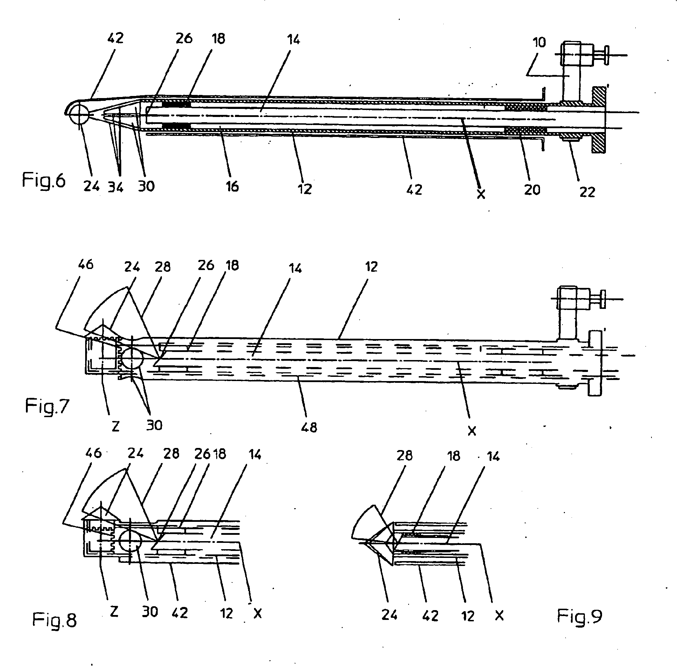

Eine weitere Abwandlung der Ausführungsform gemäß Fig. 4 ist in Fig. 6 gezeigt. Hier ist die Schutzhülse 42 in distaler Richtung weiter verlängert, so dass sie auch den Bereich der Stege 34 und des Bearbeitungswerkzeuges 24 an einer Umfangsseite umgibt. Ferner erstreckt sie sich am distalen Ende zur Hälfte über die Stirnseite des Bearbeitungswerkzeuges 24, so dass dies lediglich in beschränktem Umfangsbereich bezüglich der Längsachse X freiliegend ist und hier eine Bearbeitung vornehmen kann.

Fig. 7 und 8 zeigen Ausführungsformen, bei welchen das Bearbeitungswerkzeug 24 am distalen Ende kegelförmig und abgewinkelt ausgebildet ist. Das Bearbeitungswerkzeug 24 gemäß Fig. 7 und 8 ist als kegelförmiger Fräser- oder Schleifkörper mit ggf. entsprechender Oberflächenbeschichtung ausgebildet. Die Drehachse Z ist um 90° abgewinkelt zu der Längsachse X ausgebildet, so dass sich das Bearbeitungswerkzeug 24 gemäß Fig. 7 und 8 in radialer Richtung erstreckt. Der Antrieb des seitlich gerichteten Bearbeitungswerkzeuges 24 erfolgt hier durch eine Kegelrad- bzw. Stirnradpaarung 46. Alternativ kann diese auch als Reibradpaarung ausgebildet sein. Gehalten wird gemäß der Ausführungsform in Fig. 7 das Bearbeitungswerkzeug 34 durch eine zwischen der Optik 14. und dem Hohlschaft 12 angeordnete feststehende Hülse 48. Bei der Ausführungsform gemäß Fig. 8 wird das Bearbeitungswerkzeug 24 durch eine den Hohlschaft 12 umfänglich umgebende Schutzhülse 42 (ähnlich zu den Ausführungsbeispielen in Fig. 4 und 6) gehalten.

Bei den Ausführungsbeispielen gemäß Fig. 7 und 8 ist auch das Sichtfenster 26 bzw. das Blickfeld 28 der Endoskopoptik 14 schräg bzw. seitlich gerichtet, so dass der Bearbeitungsraum radial vor dem Bearbeitungswerkzeug 24 beobachtet werden kann.

Bei der Ausführungsform gemäß Fig. 9 ist das Bearbeitungswerkzeug 24 als kegelförmiger Fräser oder Bohrer ausgebildet, in dem über den Umfang verteilte Ausnehmungen vorgesehen sind, die zwischen den Schneiden des Fräsers platziert sind. Diese passieren das Blickfeld 28 bei Rotation periodisch, so dass ein Blick durch den Fräser hindurch, wie oben beschrieben, möglich ist. Bei der Ausführungsform gemäß Fig. 9 ist wiederum eine den Hohlschaft 12 umgebende Schutzhülse 42 vorgesehen.

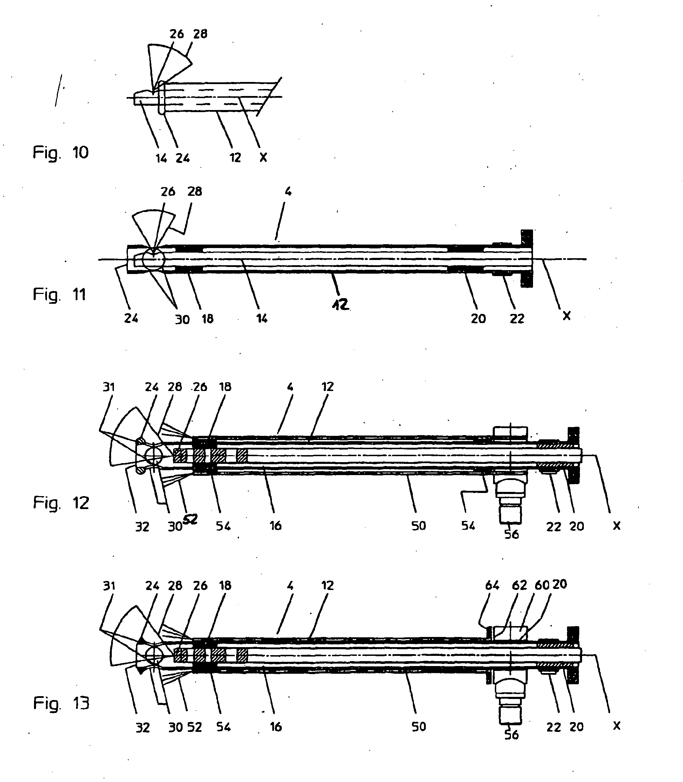

Bei der Ausführungsform gemäß Fig. 10 ist die Endoskopoptik 14 über das distale Ende des Hohlschaftes 12 distalseitig des Bearbeitungswerkzeuges 24 verlängert. Das Sichtfenster 26 ist so angeordnet, dass das Blickfeld 28 in einer retrograden Blickrichtung, d. h. in proximaler Richtung gewinkelt zu der Längsachse X gerichtet ist. D. h. das Bearbeitungswerkzeug 24 ist proximalseitig des Sichtfensters 26 angeordnet. Dies ermöglicht, durch die rückwärts gerichtete Blickrichtung 28 das Bearbeitungswerkzeug 24 im Eingriff bei der Bearbeitung zu beobachten.

Die Ausführungsform gemäß Fig. 11 entspricht im Wesentlichen der anhand von Fig. 1 beschriebenen Ausführungsform mit dem Unterschied, dass das Sichtfenster 26 der Endoskopoptik 14 umfänglich angeordnet ist, so dass ein radial gerichtetes Blickfeld 28 durch die umfänglich im Hohlschaft 12 angeordneten Ausnehmungen 30 erreicht wird.

Anhand der Fig. 12 und 13 werden zwei Ausführungsformen beschrieben, bei welchen eine zusätzliche Beleuchtungseinrichtung vorgesehen ist. Die Anordnung gemäß Fig. 12 entspricht im Wesentlichen der anhand von Fig. 1 beschriebenen Anordnung, wobei das Blickfeld in Fig. 12 leicht gewinkelt zu der Längsachse X distalwärts gerichtet ist. Der Hohlschaft 12 ist konzentrisch von einer Lichthülse 50 umgeben, welche als Lichtleiter in Richtung vom proximalen zum distalen Ende des Endoskopschaftes 4 fungiert. Dabei ist die distale Stirnseite der Lichthülse 50 als Lichtaustrittsfläche ausgebildet, aus welcher die Lichtstrahlen 52 in distaler Richtung austreten, um das Sichtfeld 28 und den Raum um das Bearbeitungswerkzeug 24 auszuleuchten. Die Lichthülse 50 ist feststehend ausgebildet. Dazu sind ebenfalls Lagerelemente 54 zwischen dem Innenumfang der Lichthülse 50 und dem Außenumfang des Hohlschaftes 12 vorgesehen, so dass der Hohlschaft 12 sich relativ zu der Lichthülse 50 drehen kann. Die Lager 54 sind vorzugsweise ebenfalls als Gleitlager ausgebildet. Am proximalen Ende der Lichthülse 50 ist ein Lichtleiteranschluss 56 vorgesehen, über welchen Licht von einer externen Lichtquelle in die Lichthülse 50 eingekoppelt werden kann.

Fig. 13 zeigt eine andere Anordnung der Lichthülse 50, bei welcher die Lichthülse 50 ebenfalls konzentrisch am Außenumfang des Hohlschaftes 12 angeordnet ist, jedoch direkt und fest mit diesem verbunden ist, so dass die Lichthülse 50 mit dem Hohlschaft 12 rotiert. Die distale Stirnseite der Lichthülse 50 ist ebenfalls als Lichtaustrittsfläche ausgestaltet, so dass Lichtstrahlen 52 in distaler Richtung austreten können, um das Sichtfeld 28 und den Umfang des Bearbeitungswerkzeuges 24 zu beleuchten. Zwischen der rotierenden Lichthülse 50 und dem Lichtleiteranschluss 56 ist eine Einkoppelstruktur 58 geschaffen, in der Weise, dass sich an den Lichtleiteranschluss 56 eine lichtleitende Hülse 60 anschließt, deren distalwärts gerichtete Stirnseite 62 als Lichtaustrittsfläche ausgebildet ist. Korrespondierend ist die proximale Stirnfläche 64 der Lichthülse 50, welche hier zusätzlich in radialer Richtung gegenüber der Lichthülse 50 erweitert ist, als Lichteintrittsfläche ausgebildet, so dass das Licht von der feststehenden Stirnfläche 62 in die rotierende Stirnfläche 64 eingestrahlt werden kann und dann über die Lichthülse 50 weiter zum distalen Ende des Endoskopschaftes 4 geleitet werden kann.

In der Ausführungsform gemäß Fig. 13 ist eine weitere mögliche Ausgestaltung des Bearbeitungswerkzeuges 24 gezeigt, welches eine spitze Umfangskante im Gegensatz zu der gerundeten Ausgestaltung gemäß Fig. 1 und 12 aufweist.

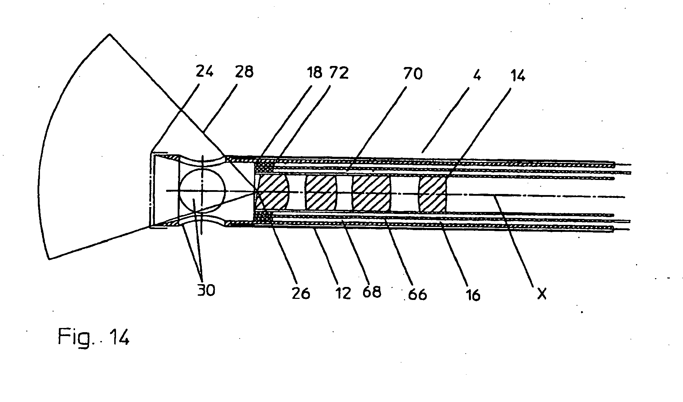

Anhand des Ausführungsbeispiels in Fig. 14 wird die mögliche Anordnung von zwei Spülkanälen zwischen der Endoskopoptik 14 und dem Hohlschaft 12 beschrieben. Das Bearbeitungswerkzeug 24, welches von der distalen Stirnkante des Hohlschaftes 12 gebildet wird und die Anordnung des Blickfeldes 28 und des Sichtfensters 26 entsprechen den vorangehend beschriebenen Ausführungsformen. Gemäß der Ausführungsform in Fig. 14 ist in dem Freiraum 16 zwischen der Endoskopoptik 14 und der Innenwandung des Hohlschaftes 12 eine Hülse 66 angeordnet, welche den Freiraum 16 in zwei ringförmige Kanäle 68 und 70 unterteilt. Die Kanäle 68 und 70 bilden zwei Spülkanäle, wobei durch einen der Kanäle beispielsweise eine Spülflüssigkeit oder ein Spülgas vom proximalen Ende her zugeführt und durch den anderen Kanal wieder abgeführt werden kann. Die Hülse 66 ist durch Distanzelemente 72 konzentrisch beabstandet zu der der Endoskopoptik 14 vorzugsweise drehfest zu dieser gehalten. Die Lagerelemente 18, welche zwischen der Hülse 66 und der Innenwandung des Hohlschaftes 12 angeordnet sind, ermöglichen eine Rotation des Hohlschaftes 12 relativ zu der Hülse 66 und halten gleichzeitig die Hülse 66 konzentrisch zu dem Hohlschaft 12.

Die vorangehend beschriebenen Ausführungsbeispiele enthalten verschiedene Elemente, welche auch in anderer Weise in Instrumenten kombiniert werden können. So können die unterschiedlich ausgestalteten Bearbeitungswerkzeuge 24 auch jeweils bei den anderen Ausführungsbeispielen Verwendung finden. Auch lassen sich Elemente wie die Lichthülse 50 entsprechend bei den anderen Ausführungsbeispielen verwenden, insbesondere auch bei dem flexiblen Endoskopschaft 4. Auch die Anordnung von zwei Spülkanälen 68 und 70, welche in Fig. 14 gezeigt ist, kann auch bei den anderen beschriebenen Ausführungsbeispielen Verwendung finden.

Ferner wurde bei den vorangehend beschriebenen Beispielen der Hohlschaft 12 immer als rotatorisch angetriebenes Bauteil beschrieben. Alternativ oder zusätzlich kann der Hohlschaft 12 auch eine translatorische, oszillierende Bewegung in Richtung der Längsachse X ausführen.

### Bezugszeichenliste

- 2: Endoskopgehäuse
- 4: Endoskopschaft
- 6: Einblicköffnung
- 8: Lichtanschluss
- 10: Antrieb
- 12: Hohlschaft
- 14: Endoskopoptik
- 16: Freiraum
- 18, 20: Lagerelemente
- 22: Ritzel
- 24: Bearbeitungswerkzeug
- 26: Sichtfenster
- 28: Blickfeld
- 30: Ausnehmungen
- 31: Stege
- 32: distale Stirnfläche
- 34: Stege
- 36: Sondenkanal
- 38: Schmirgeldraht
- 40: Vorsprung
- 42: Schutzhülse
- 44: Lagerelement
- 46: Kegelradpaarung
- 48: Hülse
- 50: Lichthülse
- 52: Lichtstrahlen
- 54: Lagerelemente
- 56: Lichtleiteranschluss
- 58: Einkoppelstruktur
- 60: Hülse
- 62, 64: Stirnflächen
- 66: Hülse
- 68, 70: Kanäle
- 72: Lager bzw. Distanzelemente
- X: Längsachse des Endoskopschaftes
- Y: Längsachse des Sondenkanals
- Z: Drehachse des Bearbeitungswerkzeuges

## Patentansprüche

1. Endoskop mit einem an dessen distalen Ende angeordneten Bearbeitungswerkzeug (24), welches mittels eines sich in Längsrichtung (X) des Endoskops erstreckenden beweglichen Hohlschaftes (12) bewegbar ist,
wobei im Inneren des beweglichen Hohlschaftes (12) eine Endoskopoptik (14) angeordnet ist und das Blickfeld (28) der Endoskopoptik (14) zumindest teilweise in radialer Richtung bezüglich der Längsachse (X) des Endoskops gerichtet ist, am **dadurch gekennzeichnet, dass** distalen Ende in der Umfangswandung des Hohlschaftes (12) mehrere Ausnehmungen (30) ausgebildet sind, welche im Blickfeld (28) der Endoskopoptik (14) liegen oder in das Blickfeld (28) der Endoskopoptik (14) bewegbar sind, um eine Beabachtung in radialer Richtung bzw, im Winkel zur Längsachse des Endoskops durch die Umfangewandung des Hohlschaftes (12) hindurch zu ermöglichen.

2. Endoskop nach Anspruch 1, **dadurch gekennzeichnet, dass** der Hohlschaft (12) in Längsrichtung (X) des Endoskops beweglich und/oder um seine Längsachse (X) drehbar ist.

3. Endoskop nach Anspruch 1 oder 2, **dadurch gekennzeichnet, dass** der Hohlschaft (12) einen kreisförmigen Querschnitt aufweist.

4. Endoskop nach einem der vorangehenden Ansprüche, **dadurch gekennzeichnet, dass** Lagerelemente (18, 20) zwischen einer Innenwandung des Hohlschaftes (12) und einer Außenwandung der Endoskopoptik (14) angeordnet sind.

5. Endoskop nach einem der vorangehenden Ansprüche, **dadurch gekennzeichnet, dass** am proximalen Ende ein Antrieb (10) zur Bewegung des Hohlschaftes (12) vorgesehen ist.

6. Endoskop nach einem der vorangehenden Ansprüche, **dadurch gekennzeichnet, dass** der Hohlschaft (12) flexibel ausgebildet ist.

7. Endoskop nach einem der vorangehenden Ansprüche, **dadurch gekennzeichnet, dass** die Endoskopoptik (14) flexibel ausgebildet ist.

8. Endoskop nach einem der vorangehenden Ansprüche, **dadurch gekennzeichnet, dass** am distalen Ende des Hohlschaftes (12) eine Kupplung zur Aufnahme des Bearbeitungswerkzeuges (24) ausgebildet ist.

9. Endoskop nach einem der vorangehenden Ansprüche, **dadurch gekennzeichnet, dass** am distalen Ende des Hohlschaftes (12) ein Getriebe (46), insbesondere ein Winkeltrieb angeordnet ist, mittels welchem das Bearbeitungswerkzeug (24) bewegbar ist.

10. Endoskop nach einem der vorangehenden Ansprüche, **dadurch gekennzeichnet, dass** an dem Hohlschaft (12) zumindest ein sich in Längsrichtung (X) des Endoskops erstreckender Sondenkanal (36) ausgebildet ist.

11. Endoskop nach einem der Ansprüche 1 bis 7, **dadurch gekennzeichnet, dass** das Bearbeitungswerkzeug (24) direkt am distalen Ende des Hohlschaftes (12) ausgebildet ist.

12. Endoskop nach einem der vorangehenden Ansprüche, **dadurch gekennzeichnet, dass** das Bearbeitungswerkzeug (24) als Säge, Fräser und/oder Schleifwerkzeug ausgebildet ist.

13. Endoskop nach einem der vorangehenden Ansprüche, **dadurch gekennzeichnet, dass** der Hohlschaft (12) abnehmbar ausgebildet ist.

14. Endoskop nach einem der vorangehenden Ansprüche, **dadurch gekennzeichnet, dass** die Endoskopoptik (14) als Linsensystem, Faseroptik oder Kamerasystem ausgebildet ist.

15. Endoskop nach einem der vorangehenden Ansprüche, **dadurch gekennzeichnet, dass** eine Bilderfassung über die Endoskopoptik (14) mittels einer Kamera erfolgt und die Bilderfassung mit der Bewegung des Hohlschaftes (12) synchronisiert ist.

16. Endoskop nach einem der vorangehenden Ansprüche, **dadurch gekennzeichnet, dass** zwischen der Außenwandung der Endoskopoptik (14) und der Innenwandung des Hohlschaftes (12) zumindest ein Spülkanal (16; 68, 70) ausgebildet ist.

17. Endoskop nach einem der vorangehenden Ansprüche, **dadurch gekennzeichnet, dass** eine Lichthülse (50) zur Beleuchtung eines das distale Ende des Endoskops umgebenden Raumes vorgesehen ist.

18. Endoskop nach Anspruch 17, **dadurch gekennzeichnet, dass** die Lichthülse (50) außerhalb des Hohlschaftes (12) angeordnet ist.

19. Endoskop nach Anspruch 17 oder 18, **dadurch gekennzeichnet, dass** die Lichthülse (50) als Lichtleiter ausgebildet ist.

20. Endoskop nach Anspruch 17 oder 18, **dadurch gekennzeichnet, dass** in der Lichthülse (50) zumindest ein Leuchtmittel angeordnet ist.

21. Endoskop nach einem der Ansprüche 17 bis 19, **dadurch gekennzeichnet, dass** die Lichthülse (50) gemeinsam mit dem Hohlschaft (12) beweglich ist.

22. Endoskop nach einem der Ansprüche 17 bis 19, **dadurch gekennzeichnet, dass** die Lichthülse (50) relativ zu dem Hohlschaft (12) beweglich ist.

23. Endoskop nach einem der vorangehenden Ansprüche, **dadurch gekennzeichnet, dass** der Hohlschaft (12) umfänglich von einer Schutzhülse (42) umgeben ist.

## Claims

1. An endoscope with a machining tool (24) which is arranged at its distal end and which may be moved by way of a movable hollow shank (12) extending in the longitudinal direction (X) of the endoscope, wherein endoscope optics (14) are arranged in the inside of the movable hollow shank (12), and the viewing field (28) of the endoscope optics (14) at least partly is directed in the radial direction with respect to the longitudinal axis (X) of the endoscope, **characterised in that** several recesses (30) are formed at the distal end on the peripheral wall of the hollow shank (12), which lie in the viewing field (28) of the endoscope optics (14) or may be moved into the viewing field (28) of the endoscope optics (14), in order to permit an observation in the radial direction or at an angle to the longitudinal axis of the endoscope through the peripheral wall of the hollow shank (12).

2. An endoscope according to claim 1, **characterised in that** the hollow shank (12) is movable in the longitudinal direction (X) of the endoscope and/or is rotatable about its longitudinal axis (X).

3. An endoscope according to claim 1 or 2, **characterised in that** the hollow shank (12) has a circular cross section.

4. An endoscope according to one of the preceding claims, **characterised in that** bearing elements (18, 20) are arranged between an inner wall of the hollow shank (12) and an outer wall of the endoscope optics (14).

5. An endoscope according to one of the preceding claims, **characterised in that** a drive (10) for the movement of the hollow shank (12) is provided at the proximal end.

6. An endoscope according to one of the preceding claims, **characterised in that** the hollow shank (12) is designed in a flexible manner.

7. An endoscope according to one of the preceding claims, **characterised in that** the endoscope optics (14) are designed in a flexible manner.

8. An endoscope according to one of the preceding claims, **characterised in that** a coupling for receiving the machining tool (24) is formed at the distal end of the hollow shank (12).

9. An endoscope according to one of the preceding claims, **characterised in that** a gear (46) in particular a right-angle gear drive is arranged at the distal end of the hollow shank (12), by way of which the machining tool (24) may be moved.

10. An endoscope according to one of the preceding claims, **characterised in that** at least one probe channel (36) extending in the longitudinal direction (X) of the endoscope is formed on the hollow shank (12).

11. An endoscope according to one of the claims 1 to 7, **characterised in that** the machining tool (24) is formed directly on the distal end of the hollow shank (12).

12. An endoscope according to one of the preceding claims, **characterised in that** the machining tool (24) is designed as a saw, miller and/or grinding tool.

13. An endoscope according to one of the preceding claims, **characterised in that** the hollow shank (12) is designed in a removable manner.

14. An endoscope according to one of the preceding claims, **characterised in that** the endoscope optics (14) are designed as a lens systems, fibre optics or camera system.

15. An endoscope according to one of the preceding claims, **characterised in that** picture detection is effected via the endoscope optics (14) by way of a camera, and the picture detection is synchronised with the movement of the hollow shank (12).

16. An endoscope according to one of the preceding claims, **characterised in that** at least one rinsing channel (16; 68, 70) is formed between the outer wall of the endoscope optics (14) and the inner wall of the hollow shank (12).

17. An endoscope according to one of the preceding claims, **characterised in that** a light sleeve (50) for illuminating a space surrounding the distal end of the endoscope is provided.

18. An endoscope according to claim 17, **characterised in that** the light sleeve (50) is arranged outside the hollow shank (12).

19. An endoscope according to claim 17 or 18, **characterised in that** the light sleeve (50) is designed as an optical waveguide.

20. An endoscope according to claim 17 or 18, **characterised in that** at least one lighting means is arranged in the light sleeve (50).

21. An endoscope according to one of the claims 17 to 19, **characterised in that** the light sleeve (50) is movable together with the hollow shank (12).

22. An endoscope according to one of the claims 17 to 19, **characterised in that** the light sleeve (50) is movable relative to the hollow shank (12).

23. An endoscope according to one of the preceding claims, **characterised in that** the hollow shank (12) is surrounded peripherally by a protective sleeve (42).

## Revendications

1. Endoscope comprenant un outil d'usinage (24) disposé à son extrémité distale, lequel est déplaçable au moyen d'une tige creuse (12) mobile, s'étendant dans la direction longitudinale (X) de l'endoscope,
une optique d'endoscope (14) étant disposée à l'intérieur de la tige creuse (12) mobile, et le champ de vision (28) de l'optique d'endoscope (14) étant orienté, au moins partiellement, en direction radiale relativement à l'axe longitudinal (X) de l'endoscope,
**caractérisé en ce qu'**à l'extrémité distale sont ménagés, dans la paroi périphérique de la tige creuse (12), plusieurs évidements (30), qui sont situés dans le champ de vision (28) de l'optique d'endoscope (14) ou peuvent être déplacés dans le champ de vision (28) de l'optique d'endoscope (14), pour permettre une observation en direction radiale ou sous un angle par rapport à l'axe longitudinal de l'endoscope, à travers la paroi périphérique de la tige creuse (12).

2. Endoscope selon la revendication 1, **caractérisé en ce que** la tige creuse (12) est mobile dans la direction longitudinale (X) de l'endoscope et/ou peut tourner autour de son axe longitudinal (X).

3. Endoscope selon la revendication 1 ou 2, **caractérisé en ce que** la tige creuse (12) présente une section transversale circulaire.

4. Endoscope selon l'une des revendications précédentes, **caractérisé en ce que** des éléments formant palier (18, 20) sont disposés entre une paroi intérieure de la tige creuse (12) et une paroi extérieure de l'optique d'endoscope (14).

5. Endoscope selon l'une des revendications précédentes, **caractérisé en ce qu'**à l'extrémité proximale est prévu un entraînement (10) pour le déplacement de la tige creuse (12).

6. Endoscope selon l'une des revendications précédentes, **caractérisé en ce que** la tige creuse (12) est dotée d'une structure flexible.

7. Endoscope selon l'une des revendications précédentes, **caractérisé en ce que** l'optique d'endoscope (14) est dotée d'une structure flexible.

8. Endoscope selon l'une des revendications précédentes, **caractérisé en ce qu'**à l'extrémité distale de la tige creuse (12) est formé un accouplement destiné à recevoir l'outil d'usinage (24).

9. Endoscope selon l'une des revendications précédentes, **caractérisé en ce qu'**à l'extrémité distale de la tige creuse (12) est disposé un engrenage (46), en particulier un engrenage d'angle, au moyen duquel l'outil d'usinage (24) peut être déplacé.

10. Endoscope selon l'une des revendications précédentes, **caractérisé en ce que** sur la tige creuse (12) est ménagé au moins un canal à sonde (36) s'étendant dans la direction longitudinale (X) de l'endoscope.

11. Endoscope selon l'une des revendications 1 à 7, **caractérisé en ce que** l'outil d'usinage (24) est formé directement à l'extrémité distale de la tige creuse (12).

12. Endoscope selon l'une des revendications précédentes, **caractérisé en ce que** l'outil d'usinage (24) est conçu sous forme d'une scie, d'une fraise et/ou d'un outil de meulage.

13. Endoscope selon l'une des revendications précédentes, **caractérisé en ce que** la tige creuse (12) est conçue démontable.

14. Endoscope selon l'une des revendications précédentes, **caractérisé en ce que** l'optique d'endoscope (14) est conçue sous forme d'un système de lentilles, d'une fibre optique ou d'un système à caméra.

15. Endoscope selon l'une des revendications précédentes, **caractérisé en ce qu'**un enregistrement d'images est effectué, au travers de l'optique d'endoscope (14), au moyen d'une caméra, et l'enregistrement d'images est synchronisé avec le déplacement de la tige creuse (12).

16. Endoscope selon l'une des revendications précédentes, **caractérisé en ce qu'**au moins un canal de lavage (16 ; 68, 70) est ménagé entre la paroi extérieure de l'optique d'endoscope (14) et la paroi intérieure de la tige creuse (12).

17. Endoscope selon l'une des revendications précédentes, **caractérisé en ce qu'**un manchon éclairant (50) est prévu pour éclairer un espace entourant l'extrémité distale de l'endoscope.

18. Endoscope selon la revendication 17, **caractérisé en ce que** le manchon éclairant (50) est disposé à l'extérieur de la tige creuse (12).

19. Endoscope selon la revendication 17 ou 18, **caractérisé en ce que** le manchon éclairant (50) est conçu sous forme d'un guide de lumière.

20. Endoscope selon la revendication 17 ou 18, **caractérisé en ce qu'**au moins un moyen d'éclairage est disposé dans le manchon éclairant (50).

21. Endoscope selon l'une des revendications 17 à 19, **caractérisé en ce que** le manchon éclairant (50) est mobile conjointement avec la tige creuse (12).

22. Endoscope selon l'une des revendications 17 à 19, **caractérisé en ce que** le manchon éclairant (50) est mobile par rapport à la tige creuse (12).

23. Endoscope selon l'une des revendications précédentes, **caractérisé en ce que** la tige creuse (12) est entourée, sur sa périphérie, par un manchon protecteur (42).
